# EUROPEAN PATENT SPECIFICATION

(11) **EP 0 750 510 B1**
(45) Date of publication and mention of the grant of the patent: **23.10.2002**
(21) Application number: 95913073.3
(22) Date of filing: 08.03.1995
(51) Int. Cl.: A61K 38/20, A61K 39/395, A61P 17/10

(54) **ANTI-ACNE SKIN TREATMENT COMPOSITION**
HAUTBEHANDLUNGSMITTEL GEGEN AKNE
COMPOSITION POUR LE TRAITEMENT DE LA PEAU ACNEIQUE

(30) Priority: 15.03.1994 GB 9405021
(43) Date of publication of application: 02.01.1997
(73) Proprietor: CAMBRIDGE BIOCLINICAL LIMITED, Uppingham, Rutland LE15 9UD (GB)
(72) Inventor: EVENSON, Alan, Franklin Lakes, NJ 07417 (US); GIBSON, Walter Thomas, Finedon, Northampton NN9 5NL (GB); GREEN, Martin Richard, Cambridge CB4 1HS (GB); GUY, Robert, Cambridge CB1 4GJ (GB); KEALEY, Terence George Evelyn, Cambridge CB1 2HN (GB)
(74) Representative: Paget, Hugh Charles Edward
(86) International application number: EP9500876
(87) International publication number: WO95024917

(56) References cited:
- WO-A-89/05653
- WO-A-93/24134
- BRITISH JOURNAL OF DERMATOLOGY, vol. 127, no. 4, October 1992 pages 305-311, KRISTENSEN M. ET AL. 'Distribution of interleukin 1 receptor antagonist protein (IRAP), interleukin 1 receptor and interleukin 1 alpha in normal and psoriatic skin ...'
- THE JOURNAL OF INVESTIGATIVE DERMATOLOGY, vol. 98, no. 6, June 1992 NEW YORK (US), pages 895-901, INGHAM E. ET AL. 'Pro-inflammatory levels of Interleukin-1alpha-Like Bioactivity Are present in the Majority of Open Comedones in Acne Vulgaris' cited in the application

## Description

The present invention relates to compositions for topical application to the skin and to their cosmetic and pharmaceutical use. In particular the invention relates to compositions suitable for use in the treatment of acne and cosmetic conditions associated with acne such as spots and pimples.

### BACKGROUND TO THE INVENTION

Acne vulgaris is a disease of the human sebaceous unit. The sebaceous pilosebaceous duct has been implicated in the aetiology of acne, with hyperproliferation, hyperkeratinisation and abnormal desquamation of the duct cells leading to comedone (or non-inflamed lesion) formation which is the primary symptom of acne. Subsequently the pilosebaceous follicle wall may rupture leading to inflammatory skin reactions.

The isolation and maintenance of the human pilosebaceous duct in-vitro has previously been described by Guy et al., British Journal of Dermatology (1993) 128 242-248. This paper further reported that the known anti-acne treatment, 13-cis retinoic acid, acts directly at the level of the duct.

The present inventors have significantly improved upon the isolated human sebaceous duct model reported previously. By maintaining the sebaceous duct in "keratinocyte serum-free medium" supplied commercially, supplemented with bovine pituitary extract and a high concentration of calcium chloride (ca 2mM), in place of supplemented William's E medium as previously reported, retention of duct architecture over a period of seven days with no fall in the rate of cell division is obtained. Furthermore, the rates of cell division in ducts maintained in keratinocyte medium have been found to be significantly higher than in ducts maintained in William's E medium after twenty four hours and seven days.

It has been reported in the literature that bioactive Interleukin-1α-like material is present in the majority of open acne comedones (Ingham et al, Journal of Investigative Dermatology (1992), 98 (6), 895-901). Furthermore, it has been suggested that this cytokine material may be involved in the initiation of inflammation in acne following rupture of the pilosebaceous follicle wall.

The present inventors have, however, found that when the isolated human pilosebaceous duct is maintained in the presence of Interleukin-1α (hereinafter IL-1α), hypercornification of the duct occurs which leads to blocking of the duct and comedone formation. This suggests that IL-1α is implicated directly in ductal hypercornification and comedone formation and not, as has been supposed previously, simply in the initiation of any subsequent inflammatory response. Treatments directed to antagonising IL-1α function are therefore useful in reducing or limiting ductal hypercornification and comedone formation which is a main symptom of acne.

### DEFINITION OF THE INVENTION

In an aspect the invention provides the use of one or more antagonists of IL-1α function for the manufacture of a medicament for the treatment of acne by reduction or limitation of hypercornification of the sebaceous duct.

In a related aspect, the invention provides a method for screening or testing candidate substances to identify substances suitable for treating acne comprising contacting the test substance with an isolated human sebaceous duct maintained in keratinocyte serum-free medium in the presence of IL-1α and assessing the response of the duct to the test substance. Substances which antagonise IL-1α function and are therefore of use in the treatment of acne will reduce or prevent ductal hypercornification.

### DISCLOSURE OF THE INVENTION

As used herein, the term "antagonist of IL-1α function" means any agent capable of interfering with the stimulatory effect of IL-1α on cell growth. In particular, it means any agent which has the effect of reducing or eliminating any changes in the properties of the isolated human pilosebaceous duct preparation, as herein described, caused by administration of IL-1α alone. Antagonists of IL-1α function include agents that interfere with the activity of IL-1α.

Suitably, IL-1α activity may be blocked by the IL-1 receptor antagonist protein (hereinafter IL-1rap).

IL-1rap is a 23-25kDa protein which may be obtained by purification from the urine of patients with monocytic leukaemia (see "The Cytokine Handbook", Chapter 3, published by Academic Press). Alternatively, IL-1rap may be prepared using conventional recombinant DNA techniques.

An alternative possibility is to use an antibody to IL-1α, to antagonise its function.

Such an antibody, which may be monoclonal or polyclonal, may be generated by techniques conventional in the art, for example by using recombinant DNA techniques. Specific binding subunits or antibody fragments may also be used. These may similarly be generated by conventional techniques such as enzymic digestion of intact antibody molecules, for example using papain or pepsin, or using recombinant DNA techniques. Antibody fragments may also be generated by conventional molecular biology techniques.

Conveniently, compositions comprise one or more antagonists of IL-1α function in an amount of from 0.000001 to 10% by weight of the composition, preferably from 0.0001 to 1% by weight, more especially from 0.0001 to 0.1% by weight of the composition.

The compositions preferably also comprise a vehicle to act as a diluent, disperser or carrier for the IL-1α antagonist in the composition so as to facilitate its distribution when the composition is applied to the skin. Preferably the vehicle is cosmetically and/or physiologically acceptable.

Vehicles can include water or substances such as liquid or solid emollients, solvents, humectants, thickeners and powders.

Examples of each of these types of vehicle, which can be used singly or as mixtures of one or more vehicles, are as follows:
Emollients, such as stearyl alcohol, glyceryl monoricinoleate, glyceryl monostearate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, tallow, lard, olive oil, palm kernel oil, rapeseed oil, safflower seed oil, evening primrose oil, soybean oil, sunflower seed oil, avocado oil, olive oil, sesame seed oil, coconut oil, arachis oil, castor oil, acetylated lanolin alcohols, petroleum jelly, mineral oil, butyl myristate, isostearic acid, palmitatic acid, isopropyl linoleate, lauryl lactate, myristyl lactate, decyl oleate, myristyl myristate;
Propellants, such as air, propane, butane, isobutane, dimethyl ether, carbon dioxide, nitrous oxide;
Solvents such as ethyl alcohol, methylene chloride, isopropanol, acetone, ethylene glycol monoethyl ether, diethylene glycol monobutyl ether, diethylene glycol monoethyl ether, dimethyl sulphoxide, dimethyl formamide, tetrahydrofuran;
Powders, such as chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

The vehicle will usually form from 10 to 99.9%, preferably from 50 to 99% by weight of the emulsion, and can, in the absence of other adjuncts, form the balance of the composition. The vehicle must be acceptable in the sense of being compatible with the other ingredients of the formulation and not deleterious to the recipient thereof.

The composition according to the inventor can also comprise other materials which are conventionally useful in cosmetic or therapeutic products for topical application to the skin, such as surfactants, for example anionic, nonionic and amphoteric surfactants, preservatives, perfumes, moisturisers and antioxidants.

### USE OF THE COMPOSITION

The composition is intended primarily as a product for topical application to human skin for treating acne, spots and pimples. References herein to treatment extend to prophylaxis as well as the treatment of established conditions.

It will be appreciated that the amount of the composition and the frequency of application to the skin will depend on the condition of the patient and the particular antagonist of IL-1α function used. In general, topical application of from 0.1µg to 10mg daily of a selected antagonist is proposed.

In use, a small quantity of the composition, for example 1ml is applied to areas of the skin from a suitable container or applicator and, if necessary, it is then spread over and/or rubbed into the skin using the hand or fingers or a suitable device.

The topical skin treatment composition may conveniently be formulated as a lotion having a viscosity of from 4,000 to 10,000 mPas, a fluid cream having a viscosity of from 10,000 to 20,000 mPas or a cream having a viscosity of from 20,000 to 100,000 mPas, or above. The composition can be packaged in a suitable container to suit its viscosity and intended use by the consumer. For example, a lotion or fluid cream can be packaged in a bottle or a roll-ball applicator or a propellant-driven aerosol device or a container fitted with a pump suitable for finger operation. When the composition is a cream, it can simply be stored in a non-deformable bottle or squeeze container, such as a tube or a lidded jar.

In order that the invention may be well understood the following examples are given by way of illustration only.

### Example 1

A lotion has the following formulation:

| | % w/w |
|---|---|
| IL-1rap | 0.01 |
| Ethanol | 10 |
| Perfume | qs |
| Butylated hydroxytoluene | 0.01 |
| Water | to 100 |

### Example 2

The effectiveness of compositions comprising an antagonist of IL-1α function was studied as follows:

### Isolation of Sebaceous Ducts

Redundant human female facial skin was obtained from cosmetic surgical procedures. Thereafter, layers of skin were removed by means of a keratome. Initially 0.1mm of the skin surface was taken, to remove the epidermal layer. A second layer of 0.2mm of dermis was then taken and placed in phosphate-buffered saline. This layer contains the pilosebaceous ducts. The ducts were easily identified using a dissecting microscope, as they are much larger than the ducts of the vellus follicle, and lack the prominent hair of the terminal follicle. In addition, the ducts were seen to contain large quantities of sebum which appeared dark on transillumination. The ducts were removed by gentle microdissection.

### Maintenance of Isolated Pilosebaceous Ducts

Ducts were maintained in keratinocyte serum free basal medium (supplied by Gibco) supplemented by 50µg/ml bovine pituitary extract, and where appropriate 1ng/ml interleukin-1α (IL-1α), at 37°C Where appropriate the antagonist of IL-1α function interleukin-1α receptor antagonist protein (hereinafter IL1rap) was added at the same time as the IL-1α at 1000ng/ml.

Optionally antibiotics and antifungal agents may be added to the culture medium to prevent bacterial and fungal contamination.

### Morphology of Ducts and Behaviour in Culture

Ducts maintained in the absence of IL-1α maintained normal morphology for at least 7 days. The duct shows an organised stratified keratinised squamous epithelium similar to that seen in tissue sections both on isolation and at the end of the culture period. (For an example of normal duct morphology see Figure 3 of Guy et al. British Journal of Dermatol. 1993 128, 242-248). However on the addition of IL-1α to the culture medium typically at 1ng/ml, the normal duct morphology was lost over 7 days. The duct hypercornified with many keratinised squames filling the lumen of the duct. This process resembles the features of duct blockage and comedone formation that occurs in vivo.

Addition of the antagonist to IL-1α function (IL1rap) e.g. at 1000ng/ml prevented the hypercornification of the duct, with maintenance of normal morphology. Furthermore addition of a neutralising antibody to interleukin-1α (e.g. at 1 microgram/ml) in the presence of interleukin-1α also prevented the hypercornification of the duct. These data show that antagonists of IL-1α function, including antagonists that can inhibit receptor function or antagonists that can prevent ligand function can prevent duct hypercornification and blockage in vitro in response to IL-1α.

The maintenance of the duct described here over 7 days is vastly superior to that reported by Guy et al (same reference as above) using the different medium, Williams E.

## Claims

1. Use of an antagonist of Interleukin-1α function in the manufacture of a composition for the treatment of acne by reduction or limitation of hypercornification of the sebaceous duct.

2. Use according to claim 1 wherein the antagonist of Interleukin-1α function comprises Interleukin-1 receptor antagonist protein.

3. Use according to claim 1 wherein the antagonist of Interleukin-1α function comprises an antibody to Interleukin-1α.

4. Use according to any one of claims 1 to 3 wherein the antagonist of Interleukin-1α function is present in an amount of from 0.000001 to 10% by weight of the composition.

5. Use according to claim 4 wherein the antagonist of Interleukin-1α function is present in an amount of from 0.00001 to 10% by weight of the composition.

6. Use according to any one of claims 1 to 6 further comprising including in the composition a cosmetically or physiologically acceptable vehicle.

7. Use according to claim 6 wherein the vehicle is a diluent, dispenser or carrier for the antagonist which facilitates its distribution when the composition is applied to the skin.

8. A method of testing a substance for its ability to reduce or limit hypercornification of the sebaceous duct, comprising the steps of:
(i) contacting the test substance with an isolated human sebaceous duct maintained in a keratinocyte serum-free medium, in the presence of Interleukin-1α; and
(ii) assessing the response of the duct to the test substance.

## Patentansprüche

1. Verwendung eines Antagonisten der Interleukin-1α-Funktion bei der Herstellung einer Zusammensetzung zur Behandlung von Akne durch die Reduktion oder Begrenzung von übermäßiger Verhornung des Talgkanals.

2. Verwendung nach Anspruch 1, worin der Antagonist der Interleukin-1α-Funktion Interleukin-1-Rezeptor-Antagonist-Protein umfasst.

3. Verwendung nach Anspruch 1, worin der Antagonist der Interleukin-1α-Funktion einen Antikörper gegen Interleukin-1α umfasst.

4. Verwendung nach einem der Ansprüche 1 bis 3, worin der Antagonist der Inter-Ieukin-1α-Funktion in einer Menge von 0,000001 bis 10 Gew.-% der Zusammensetzung enthalten ist.

5. Verwendung nach Anspruch 4, worin der Antagonist der Interleukin-1α-Funktion in einer Menge von 0,00001 bis 10 Gew.-% der Zusammensetzung enthalten ist.

6. Verwendung nach einem der Ansprüche 1 bis 6, weiters umfassend das Miteinbeziehen eines kosmetisch oder physiologisch annehmbaren Vehikels in die Zusammensetzung.

7. Verwendung nach Anspruch 6, worin das Vehikel ein Verdünner, ein Dispergator oder ein Träger für den Antagonisten ist, der seine Abgabe erleichtert, wenn die Zusammensetzung auf die Haut aufgetragen wird.

8. Verfahren zum Testen einer Substanz auf ihre Fähigkeit, übermäßige Verhornung des Talgkanals zu verringern oder zu begrenzen, folgende Schritte umfassend:
(i) das Kontaktieren der Testsubstanz mit einem isolierten menschlichen Talgkanal, der in einem serumfreien Keratinozyten-Medium gehalten wird, in Gegenwart von Interleukin-1α; und
(ii) das Bestimmen des Reaktion des Kanals auf die Testsubstanz.

## Revendications

1. Utilisation d'un antagoniste de la fonction d'interleukine-1α dans la fabrication d'une composition pour le traitement de l'acnée par réduction ou limitation de l'hypercornification du conduit sébacé.

2. Utilisation selon la revendication 1 où l'antanogiste de la fonction de l'interleukine-1α comprend une protéine antagoniste du récepteur d'interleukine-1.

3. Utilisation selon la revendication 1 où l'antagoniste de la fonction de l'interleukine-1α comprend un anticorps à l'interleukine-1α.

4. Utilisation selon l'une quelconque des revendications 1 à 3 où l'antagoniste de la fonction de l'interleukine-1α est présent en une quantité de 0,000001 à 10% en poids de la composition.

5. Utilisation selon la revendication 4 où l'antagoniste de la fonction d'interleukine-1α est présent en une quantité de 0,00001 à 10% en poids de la composition.

6. Utilisation selon l'une quelconque des revendications 1 à 6, comprenant de plus l'inclusion dans la composition d'un véhicule cosmétiquement ou physiologiquement acceptable.

7. Utilisation selon la revendication 6, où le véhicule est un diluant, distributeur ou support pour l'antagoniste, qui facilite sa distribution quand la composition est appliquée à la peau.

8. Méthode de test d'une substance pour son aptitude à réduire ou limiter l'hypercornification de la gaine sébacée, comprenant les étapes de:
(i) mettre en contact la substance de test avec une gaine sébacée humaine isolée maintenue dans un milieu sans sérum des kératinocytes en présence de l'interleukine 1α; et
(ii) évaluer la réponse de la gaine à la substance de test.
